# EUROPÄISCHE PATENTSCHRIFT

(11) **EP 1 320 326 B1**
(45) Veröffentlichungstag und Bekanntmachung des Hinweises auf die Patenterteilung: **24.08.2005**
(21) Anmeldenummer: 01978170.7
(22) Anmeldetag: 20.09.2001
(51) Int. Cl.: A61B 6/03

(54) **EINRICHTUNG ZUR KÜHLUNG EINER FLÄCHE, DIE UM EINE DREHACHSE ROTIERT UND DER DREHACHSE ZUGEKEHRT IST**
DEVICE FOR COOLING A SURFACE THAT ROTATES ABOUT A ROTATION AXIS AND THAT FACES THE ROTATION AXIS
DISPOSITIF POUR REFROIDIR UNE SURFACE QUI EST EN ROTATION AUTOUR D'UN AXE DE ROTATION ET EST TOURNEE VERS CET AXE DE ROTATION

(30) Priorität: 29.09.2000 DE 10048488
(43) Veröffentlichungstag der Anmeldung: 25.06.2003
(73) Patentinhaber: SIEMENS AKTIENGESELLSCHAFT, 80333 München (DE)
(72) Erfinder: SCHLÖGL, Andreas, 85521 Ottobrunn (DE); TICHY, Peter, 91080 Uttenreuth (DE); WOLFGANG, Eckhard, 81379 München (DE)
(86) Internationale Anmeldenummer: PCT/DE2001/003641
(87) Internationale Veröffentlichungsnummer: WO 2002/026132

(56) Entgegenhaltungen:
- DE-A- 19 945 415
- US-A- 5 299 249
- PATENT ABSTRACTS OF JAPAN vol. 006, no. 264 (E-150), 23. Dezember 1982 (1982-12-23) & JP 57 162248 A (HITACHI SEISAKUSHO KK), 6. Oktober 1982 (1982-10-06)

## Beschreibung

Die Erfindung betrifft eine Einrichtung zur Kühlung einer Fläche, die um eine Drehachse rotiert und der Drehachse zugekehrt ist.

Ein Beispiel einer zu kühlenden Fläche, die um eine Drehachse rotiert und der Drehachse zugekehrt ist, ist eine heiß werdende Fläche einer elektronischen Einrichtung zur elektrischen Energieversorgung der Röntgenstrahlquelle eines Computer-Tomographen, die zusammen mit der Röntgenstrahlquelle um eine Röhre zur Aufnahme eines zu untersuchenden Patienten, rotiert. So ein System wird auch als "Gantry" bezeichnet.

Bei einem solchen Computer-Tomographen wird eine möglichst hohe Rotationsfrequenz angestrebt, da damit einerseits die Anzahl der pro Zeiteinheit durchgeführten Tomographieuntersuchungen erhöht werden kann und andererseits Aufnahmen von sich schnell bewegenden Organen (z.B. Herz) artefaktfrei möglich werden.

Die begrenzende Größe für die maximal erreichbare Rotationsfrequenz ist die in Rotation zu bringende Gesamtmasse, welche neben dem Röntgenstrahlendetektor u.a. die Masse der Röntgenstrahlquelle, die Masse der Einrichtung zur elektrischen Energieversorgung der Röntgenstrahlquelle und die Masse einer mit der Röntgenstrahlquelle mitrotierenden Einrichtung zur Kühlung einer heiß werdenden Fläche der Einrichtung zur elektrischen Energieversorgung umfasst. Je geringer diese Gesamtmasse ist, desto größer ist die maximal erreichbare Rotationsfrequenz. Gleichzeitig kann durch Reduktion der Baugröße von Komponenten der Rotationsradius reduziert werden, so dass sich insgesamt eine Verringerung der Fliehkraft ergibt.

Eine Verringerung jeder zur Gesamtmasse beitragenden einzelnen Masse für sich bewirkt bereits eine Verringerung der Gesamtmasse, so auch eine Verringerung der Masse der Einrichtung zur Kühlung einer heiß werdenden Fläche der Einrichtung zur elektrischen Energieversorgung der Röntgenstrahlquelle.

Die im Anspruch 1 angegebene Erfindung stellt vorteilhafterweise eine Einrichtung zur Kühlung einer um eine Achse rotierenden und der Achse zugekehrten Fläche bereit, die mit der Fläche mitrotiert und einen massearmen Aufbau aufweist.

Die erfindungsgemäße Einrichtung weist im Wesentlichen auf: Einen Behälter für ein flüssiges Kühlmittel, der zwischen der Achse und der zu kühlenden Fläche zu einer Mitrotation mit Fläche angeordnet ist, und mindestens eine der Fläche zugekehrte Zerstäubungsdüse des Behälters, aus der das Kühlmittel während der Rotation des Behälters aufgrund der auf das Kühlmittel im Behälter wirkenden Fliehkraft als ein Zerstäubungsstrahl austritt, der auf die Fläche trifft.

Die erfindungsgemäße Einrichtung nutzt geschickt die Rotation zur Anwendung eines effizienten Zerstäubungs-Kühlverfahrens aus. Insbesondere kann das hochwirksame Verfahren des bekannten "Spray Coolings" mit allen seinen Vorzügen eingesetzt werden, wobei die Fliehkraft vorteilhaft als "Kompressor" genutzt wird. Die Fliehkraft wirkt auf das Kühlmittel im rotierenden Behälter und erzeugt einen genügend hohen Druck, der ausreicht, um das Kühlmittel durch die Zerstäubungsdüse auf die zu kühlende Fläche zu sprühen.

Die erfindungsgemäße Einrichtung ist vorteilhafterweise generell überall dort anwendbar, wo eine um eine Achse rotierende und der Achse zugekehrte Fläche zu kühlen ist, und erlaubt dabei wegen ihrer Massearmut vorteilhafterweise eine hohe maximal erreichbare Rotationsfrequenz bzw. eine Steigerung der Rotations- oder Umlauffrequenz gegenüber der Standardtechnik aufgrund der Reduktion der Fliehkraft

Eine vorteilhafte Ausgestaltung der erfindungsgemäßen Einrichtung ist so ausgebildet bzw. könnte so ausgebildet sein , dass der Zerstäubungsstrahl nicht senkrecht, sondern schräg in einem Winkel auf die zu kühlende Fläche trifft. Dadurch kann eine optimale Homogenität des auf die zu kühlende Fläche gesprühten Kühlmittels und dadurch eine verbesserte Kühlung erhalten/erreicht werden.

Bei einer vorteilhaften Ausführungsform dieser Ausgestaltung ist der Winkel, unter dem der Zerstäubungsstrahl schräg auf die zu kühlende Fläche trifft, abhängig von der Rotationsfrequenz dieser Fläche einstellbar.

Ein schräg in einem Winkel auf die zu kühlende Fläche treffender Zerstäubungsstrahl entsteht beispielsweise, wenn der Zerstäubungsstrahl mit einer senkrecht auf die Fläche gerichteten Strahlachse aus der Zerstäubungsdüse austritt. Der Zerstäubungsstrahl wird auf der Strecke von der Düse zur zu kühlenden Fläche aufgrund der Wirkung der Coriolis-Kraft in der Ebene der Rotation gekrümmt und trifft schräg in einem Winkel auf die Fläche. Der Betrag dieses Winkels hängt von der Rotationsfrequenz ab und ist umso größer , je größer die Rotationsfrequenz ist.

Unabhängig davon kann ein schräg in einem Winkel auf die zu kühlende Fläche treffender Zerstäubungsstrahl dadurch erzeugt werden, dass der Zerstäubungsstrahl mit einer derart schräg in einem Winkel zur zu kühlenden Fläche gerichteten Strahlachse aus der Zerstäubungsdüse austritt, dass der Zerstäubungsstrahl schräg in einem Winkel auf die Fläche trifft. In diesem Fall kann beispielsweise der Winkel, unter dem der Zerstäubungsstrahl schräg auf die zu kühlende Fläche trifft, durch den Winkel der Strahlachse relativ zu dieser Fläche eingestellt werden. Letztgenannter Winkel kann abhängig von der Rotationsfrequenz der Fläche einstellt werden/sein.

Eine vorteilhafte Weiterbildung der erfindungsgemäßen Einrichtung weist eine geschlossene Kühlmittelkreislaufeinrichtung auf, in welcher das zerstäubte Kühlmittel nach dem Auftreffen auf die zu kühlende Fläche gesammelt und in den Behälter rückgeführt wird.

Bei einer bevorzugten und vorteilhaften Ausgestaltung dieser Weiterbildung weist die Kühlmittelkreislaufeinrichtung eine Kühlfläche auf, an der Kühlmittel, das an der zu kühlenden Fläche verdampft ist, kondensiert.

Bei einer besonders bevorzugten und vorteilhaften Ausgestaltung der erfindungsgemäßen Einrichtung ist die zu kühlende Fläche eine heiß werdende Fläche einer um eine Röhre zur Aufnahme eines zu untersuchenden Patienten rotierenden elektronischen Einrichtung zur elektrischen Energieversorgung der Röntgenstrahlquelle eines Computer-Tomographen.

Als weitere Anwendung ist auch denkbar, solch eine Kühlungseinrichtung zur Kühlung des Targets, d.h. der Anode der Röntgenstrahlquelle selbst in ähnlicher Weise zu nutzen. Demgemäß ist eine vorteilhafte Ausführungsform der erfindungsgemäßen Einrichtung so ausgebildet, dass die zu kühlende Fläche eine heiß werdende Fläche eines Targets einer um eine Röhre zur Aufnahme eines zu untersuchenden Patienten rotierenden Röntgenstrahlquelle eines Computer-Tomographen ist.

Vorteile jeder dieser letztgenannten Einrichtungen sind:
- Sie kann Wärme hocheffizient von der zu kühlenden Fläche der Einrichtung zur elektrischen Energieversorgung der Röntgenstrahlquelle und/oder der Röntgenstrahlquelle selbst abführen;
- ihre Massearmut vermindert die in Rotation zu setzende Gesamtmasse des Tomographen (rotierender Teil der Gantry) und ermöglicht eine erwünschte höhere maximal erreichbare Rotationsfrequenz, welche die Effizienz des Tomographen durch Erhöhung der pro Zeiteinheit durchführbaren Tomographieuntersuchungen erhöht bzw. die Darstellung schnell bewegter Organe artefaktfrei ermöglicht;
- sie erfordert nur, dass die Einrichtung zur elektrischen Energieversorgung der Röntgenstrahlquelle so anzuordnen ist, dass deren zu kühlende Fläche der Rotationsachse zugekehrt ist;
- sie erlaubt den Aufbau der Einrichtung zur elektrischen Energieversorgung der Röntgenstrahlquelle mit Leistungselektronik-Bauelementen, wodurch sich die in Rotation zu setzende Gesamtmasse des Tomographen noch einmal verringert und die maximal erreichbare Rotationsfrequenz noch einmal gesteigert wird, und wodurch die Baugröße des rotierenden Teils des Tomographen und der Rotationsradius beträchtlich verringert wird.

Die Erfindung wird in der nachfolgenden Beschreibung anhand der Zeichnungen beispielhaft näher erläutert: Es zeigen:

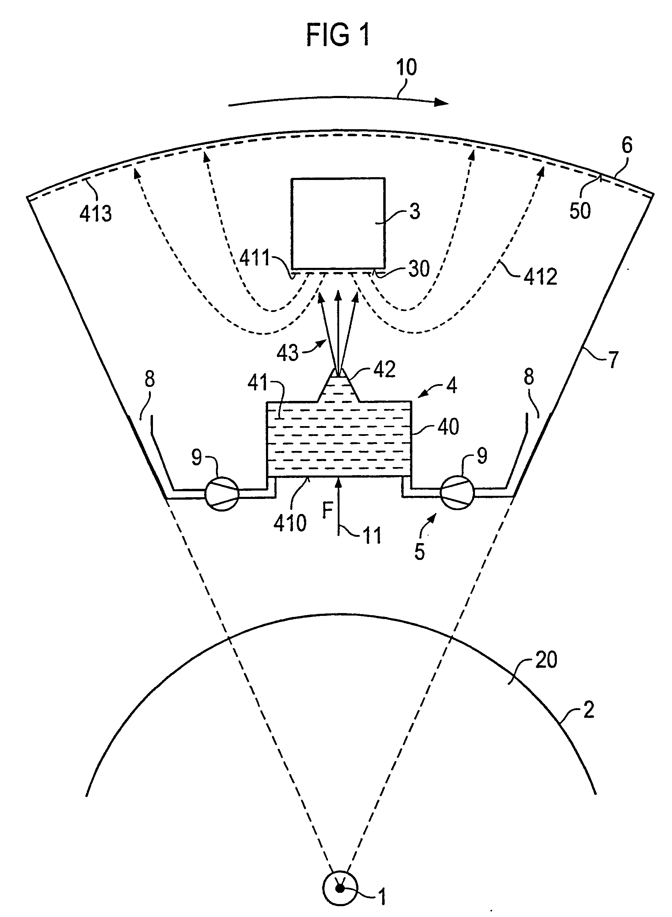

- Figur 1: eine schematische Darstellung eines Ausführungsbeispiels einer erfindungsgemäßen Einrichtung zur Kühlung einer auf dem rotierenden Teil der Gantry eines Computer-Tomographen befestigten und mit diesem Teil mitrotierenden heiß werdenden Fläche der Gantry,

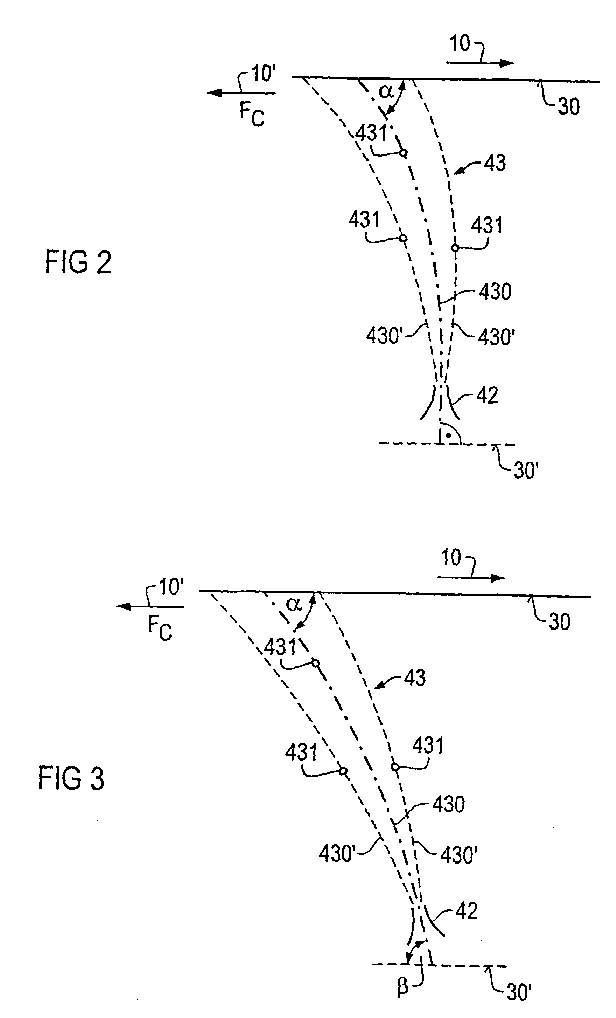

- Figur 2: eine schematische Darstellung, die den aus einer Zerstäubungsdüse des Ausführungsbeispiels der erfindungsgemäßen Einrichtung mit einer zur zu kühlenden Fläche senkrechten Strahlachse austretenden Zerstäubungsstrahls zeigt, der aufgrund der einwirkenden Coriolis-Kraft schräg in einem Winkel auf die Fläche trifft, und
- Figur 3: eine schematische Darstellung, die den aus der Zerstäubungsdüse des Ausführungsbeispiels der erfindungsgemäßen Einrichtung mit einer schräg in einem Winkel zur zu kühlenden Fläche gerichteten Strahlachse austretenden Zerstäubungsstrahls zeigt, der schräg in einem Winkel auf die Fläche trifft.

Die Figuren sind nicht maßstäblich.

Der in der Figur 1 dargestellte Teil der Gantry eines beispielhaften Computer-Tomographen weist eine Achse 1 auf, die senkrecht zur Zeichenebene der Figur 1 steht, und um die sich der rotierende Teil der Gantry des Tomographen dreht.

Die Achse 1 ist zugleich eine Längsachse der nur angedeuteten stationären Röhre 2 zur Aufnahme eines Patienten der Gantry. Der aufgenommene Patient wird im Innenraum 20 der Röhre 2 entlang der sich ebenfalls im Innenraum 20 befindenden Achse 1 verschoben.

Der um die Achse 1 und die Röhre 2 rotierende Teil der Gantry des Tomographen umfasst u.a. eine Röntgenstrahlquelle, den Röntgenstrahlendetektor, eine elektronische Einrichtung zur elektrischen Energieversorgung der Röntgenstrahlquelle und eine Einrichtung zur Kühlung einer zu kühlenden Fläche einer heiß werdenden Einrichtung des rotierenden Teils der Gantry. Die heiß werdende Einrichtung ist in der Figur 1 symbolisch durch ein mit 3 bezeichnetes Kästchen dargestellt. Sie ist auf dem rotierenden Teil der Gantry befestigt ist und rotiert synchron mit diesem Teil um die Achse 1 und die Röhre 2 mit, beispielsweise in Richtung des Pfeils 10.

Die heiß werdende Einrichtung 3 ist beispielsweise die vorzugsweise mit Halbleiterbauelementen aufgebaute Einrichtung zur elektrischen Energieversorgung der Röntgenstrahlquelle, wobei die zu kühlende Fläche eine der Achse 1 zugekehrte heiß werdende Fläche dieser Einrichtung ist, und/oder die Röntgenstrahlquelle selbst, wobei die zu kühlende Fläche eine der Achse 1 zugekehrte heiß werdende Fläche der Anode dieser Quelle, vorzugsweise eine rückseitige Fläche der geerdeten Anode, ist.

In der Figur 1 ist die zu kühlende Fläche der Einrichtung 3 symbolisch durch der Achse 1 zugekehrte Seitenfläche des Kästchens dargestellt, die mit 30 bezeichnet ist. Die zu kühlende Fläche 30 steht beispielsweise senkrecht zur Zeichenebene der Figur 1.

Die Einrichtung zur Kühlung der zu kühlenden Fläche 30 ist mit 4 bezeichnet und zwischen der Röhre 2 und der zu kühlenden Fläche 30 der Einrichtung 3 angeordnet.

Erfindungsgemäß weist die Einrichtung 4 zur Kühlung der Fläche 30 der Einrichtung 3 auf: Einen Behälter 40 für ein flüssiges Kühlmittel 41, der zwischen der Röhre 2 und der Fläche 30 der Einrichtung 3 zu einer Mitrotation mit der Einrichtung 3 und der Fläche 30 angeordnet ist, und mindestens eine der Fläche 30 zugekehrte Zerstäubungsdüse 42 des Behälters 40, aus der das Kühlmittel 41 während der Rotation des Behälters 40 aufgrund der auf das Kühlmittel 41 im Behälter 40 wirkenden Fliehkraft F als ein Zerstäubungsstrahl 43 austritt, der auf die Fläche 30 der Einrichtung 3 trifft.

Da der Behälter 40 zwischen der Röhre 2 und der Fläche 30 der Einrichtung 3 angeordnet ist und sich die Achse 1 im Innenraum 20 der Röhre 2 befindet, ist der Behälter 40 zwangsläufig zwischen der Achse 1 und der dieser Achse 1 zugekehrten und zu kühlenden Fläche 30 der Einrichtung 3 angeordnet, worauf es letztendlich ankommt.

Die Mitrotation des Behälters 40 mit der Einrichtung 3 und der Fläche 30 bedeutet, dass sich der Behälter 40 synchron mit der Einrichtung 3 und der Fläche 30 in Richtung des Pfeils 10 um die Achse 1 dreht, beispielsweise dadurch, dass der Behälter 40 und die Einrichtung 3 fest miteinander verbunden sind.

Die während der Rotation des Behälters 40 um die Achse 1 erzeugte und auf das Kühlmittel 41 im Behälter 40 wirkende Fliehkraft F wirkt in Richtung radial von der Achse 1 fort, d.h. in Richtung des Pfeils 11. Der Betrag der Fliehkraft F ist durch |**F**| = m·r·ω², wobei m die Masse des Kühlmittels 41, r den radialen Abstand des Kühlmittels 41 von der Achse 1 während der Rotation und ω die Winkelgeschwindigkeit der Rotation bedeuten.

Die senkrecht auf die der Achse 1 zugekehrte Oberfläche 410 des flüssigen Kühlmittels 41 im Behälter 40 wirkende Fliehkraft F erzeugt im Kühlmittel 41 einen hydrostatischen Druck, der bewirkt, dass das Kühlmittel 41 als der Zerstäubungsstrahl 43 aus der Zerstäubungsdüse 42 austritt, durch den das Kühlmittel 41 in Form kleiner Kühlmittelpartikel auf die zu kühlende Fläche 30 der Einrichtung 3 gesprüht wird.

Aufgrund der Rotation wirkt auf jedes sich von der Zerstäubungsdüse 42 zur Fläche 30 bewegende Kühlmittelpartikel des Zerstäubungsstrahls 43 bekanntermaßen die Coriolis-Kraft, die entgegengesetzt zur Richtung des Pfeils 10 gerichtet ist und das Kühlmittelpartikel in dieser entgegengesetzten Richtung ablenkt. Dadurch wird der Zerstäubungsstrahl 43, genaugenommen eine Strahlachse des Zerstäubungsstrahls 43 auf der Strecke zwischen der Zerstäubungsdüse 42 und der zu kühlenden Fläche 30 gekrümmt.

In der Figur 2 ist dies etwas übertrieben dargestellt. Beispielsweise trete der Zerstäubungsstrahl 43 mit einer senkrecht zur zu kühlenden Fläche 30 gerichteten Strahlachse 430 aus der Zerstäubungsdüse 42 aus. Dies ist in der Figur 2 dadurch kenntlich gemacht, dass die durch eine strichpunktierte Linie dargestellte Strahlachse 430 im Bereich der Zerstäubungsdüse 42 senkrecht zu einer gedachten, durch eine gestrichelte Linie dargestellten Fläche 30' ist, die parallel zu der senkrecht zur Zeichenebene der Figur 2 stehenden zu kühlenden Fläche 30 ist.

Ein auf der Strahlachse 430 sich bewegendes und aus der Zerstäubungsdüse 42 austretendes Kühlmittelpartikel 431 bewegt sich auf dem Weg zur Fläche 30 nicht geradlinig weiter, sondern wird infolge der einwirkenden Coriolis-Kraft **F**_{**c**}, die in Richtung des zum Pfeil 10 entgegengesetzten Pfeils 10' wirkt, abgelenkt und bewegt sich auf einer in der Figur 2 nach links gekrümmten Bahn, welche die nunmehr gekrümmte Strahlachse bzw. Strahltrajektorie 430 des Zerstäubungsstrahls 43 definiert.

Entsprechend bewegen sich (?)nicht auf der Strahlachse 430 befindliche Kühlmittelpartikel 431 des Zerstäubungsstrahls 43 seitlich der Strahlachse 430 auf gekrümmten Bahnen zur Fläche 30, wie dies durch gestrichelte Linien 430' angedeutet ist. Somit ist der Zerstäubungsstrahl 43 insgesamt gekrümmt und trifft schräg in einem Winkel auf diese Fläche 30. Dieser Winkel ist in der Figur 2 durch den Winkel α < 90° zwischen der auf die Fläche 30 treffenden Strahlachse 430 und dieser Fläche 30 dargestellt.

Ein schräges Auftreffen des Zerstäubungsstrahls 43 auf die Fläche 30 begünstigt die Erzielung einer optimalen Homogenität des auf die Fläche 30 gesprühten Kühlmittels 41,da der von den sich bewegenden Kühlmittelteilchen 431 jeweils auf die Fläche 30 übertragene Impuls nicht genau radial ausgerichtet ist, sondern auch eine zur Fläche 30 parallele Komponente aufweist, die ein Verfließen des aufgesprühten Kühlmittels in Richtung dieser Komponente begünstigt.

Der Winkel α hängt von der Winkelgeschwindigkeit ω der Rotation und damit von der Rotationsfrequenz ab. Ist diese zur Erzielung eines ausreichend kleinen Winkels α <90°ungeeignet, so kann dadurch nachgeholfen werden, dass der Zerstäubungsstrahl 43 mit einer schräg in einem Winkel zur zu kühlenden Fläche 30 gerichteten Strahlachse 430 aus der Zerstäubungsdüse 42 austritt.

In der Figur 3 ist ein solcher Fall dargestellt. Die Figur 3 unterscheidet sich von der Figur 2 lediglich darin, dass die Strahlachse 430 im Bereich der Zerstäubungsdüse 42 entgegen der Drehrichtung 10 schräg in einem Winkel β < 90° nach links zur Ebene 30 geneigt ist. Je kleiner der Winkel β gewählt ist, desto kleiner wird der Winkel α.

Der Winkel α kann in diesem Fall durch Einstellen des Winkels β abhängig von der Rotationsfrequenz eingestellt werden, insbesondere auf einen stets für die Kühlung der Fläche 30 optimalen Wert.

Anstelle nur einer Zerstäubungsdüse 42 können auch zwei oder mehrere Zerstäubungsdüsen 42 vorhanden sein, deren Zerstäubungsstrahlen 43 die Fläche 30 insbesondere schräg aus verschiedenen Richtungen treffen.

Die zu kühlende Fläche 30 kann beispielsweise auch schräg zu einer radial ausgerichteten Zerstäubungsdüse 42 angeordnet sein und muss überdies nicht eben sein, sondern kann konvex und/oder konkav gekrümmt sein.

Die Einrichtung 4 nach Figur 1 zur Kühlung der Fläche 30 der Einrichtung 3 weist eine geschlossene Kühlmittelkreislaufeinrichtung 5 auf, in welcher das zerstäubte Kühlmittel 41 nach dem Auftreffen auf die zu kühlende Fläche 30 gesammelt und in den Behälter 40 rückgeführt wird. Insbesondere weist diese Kühlmittelkreislaufeinrichtung 5 eine Kühlfläche 50 auf, an der Kühlmittel 41, das an der zu kühlenden Fläche 30 verdampft ist, kondensiert.

Die Einrichtung arbeitet beispielsweise wie folgt: Das ständig auf die zu kühlende Fläche 30 aufgesprühte Kühlmittel 41 bildet auf dieser Fläche 30 einen gestrichelt angedeuteten Kühlmittelfilm 411, aus dem ständig Kühlmittel 41 verdampft.

Der daraus resultierende und durch gestrichelte Pfeile angedeutete Kühlmitteldampf 412 bewegt sich infolge der Fliehkraft F radial von der Achse 1 fort, was durch die Krümmung der gestrichelten Pfeile angedeutet ist, und gelangt zu einer die rotierenden Teile des Tomographen radial nach außen abschließenden, mitrotierenden und vorzugsweise um die Achse 1 gebogenen Wand 6, die beispielsweise durch einen während der Rotation von selbst entstehenden Luftstrom von außen gekühlt wird.

Die der Achse 1 zugekehrte Fläche der Wand bildet die Kühlfläche 50, zu welcher der Kühlmitteldampf 412 gelangt und auf der sich der Kühlmitteldampf 412 als Kühlmittelkondensat abscheidet, das einen gleichmäßig über die ganze Kühlfläche 50 verteilten Kühlmittelfilm 413 bildet, den die Fliehkraft F gegen die Kühlfläche 50 drückt.

Durch Herunterfahren der Rotationsfrequenz könnte das im Kühlmittelfilm 413 enthaltene Kühlmittel 41 beispielsweise über Seitenwände 7 in Richtung zur Achse 1 automatisch abfließen, in Auffangtrichtern 8 aufgefangen und von den Auffangtrichtern 8 in den Behälter 40 zurückgebracht werden.

Das Zurückbringen des aufgefangenen Kühlmittels 41 kann, falls erforderlich, z.B. bei stetiger Rotation der Gantry, durch Pumpen 9 unterstützt werden. Allerdings verkomplizieren solche Pumpen 9 den Aufbau der Einrichtung 4 und vergrößern die in Rotation zu setzende Masse.

Als Kühlmittel 41 wird vorzugsweise ein inertes Kühlmittel verwendet, das chemisch nicht mit den Teilen reagiert, mit denen es in Berührung kommt. Beispielsweise kann Fluorinert verwendet werden.

In der Standardtechnik ist die Einrichtung 3 zur elektrischen Energieversorgung der Röntgenstrahlquelle des Tomographen mit standardmäßig verfügbaren Komponenten und Leistungselektronikmodulen aufgebaut, die relativ schwer sind und ein geringes Verhältnis von Chip- zu Gehäusemasse aufweisen.

Die Kühlung erfolgt durch aktive Luft- oder Wasserkühlung, wobei die Wasserkühlung effektiver arbeitet, aber auch einen höheren technischen Aufwand bedeutet und mit größeren Massen verbunden ist. Aber auch die Luftkühlung bedeutet durch das notwendige Wärmespeichervermögen des Kühlkörpers erhebliches Volumen und damit auch Gewicht.

Bei dem mit der erfindungsgemäßen Einrichtung 4 zur Kühlung der Fläche 30 der Einrichtung 3 ausgerüsteten neuartigen Tomographen besteht überdies die Möglichkeit, dass die Einrichtung 3 vorteilhafterweise nicht die relativ schweren und ein geringes Verhältnis von Chip- zu Gehäusemasse aufweisenden Kühlkomponenten und Module aufweist, sondern Leistungselektronik-Bauelemente in Leichtbauweise, wodurch die in Rotation zu setzende Masse des Tomographen noch einmal verringert und der Aufbau des rotierenden Teils des Tomographen weiter vereinfacht werden kann.

Die erfindungsgemäße Einrichtung zur Kühlung einer Fläche, die um eine Drehachse rotiert und der Drehachse zugekehrt ist, ist nicht auf die Anwendung in einem Tomographen beschränkt.

## Patentansprüche

1. Einrichtung (4) zur Kühlung einer Fläche (30), die um eine Achse (1) rotiert und der Achse (1) zugekehrt ist,
- mit einem Behälter (40) für ein flüssiges Kühlmittel (41), der zwischen der Drehachse (1) und der Fläche (30) zu einer Mitrotation mit der Fläche (30) angeordnet ist, und
- mit mindestens einer der Fläche (30) zugekehrten Zerstäubungsdüse (42) des Behälters (40), aus der das Kühlmittel (41) während der Rotation des Behälters (40) aufgrund der auf das Kühlmittel (41) im Behälter (40) wirkenden Fliehkraft (F) als ein Zerstäubungsstrahl (43) austritt, der auf die Fläche (30) trifft.

2. Einrichtung nach Anspruch 1, wobei der Zerstäubungsstrahl (43) schräg in einem Winkel (α) auf die zu kühlende Fläche (30) trifft.

3. Einrichtung nach Anspruch 2, wobei der Winkel (α), unter dem der Zerstäubungsstrahl (43) schräg auf die zu kühlende Fläche (30) trifft, abhängig von der Rotationsfrequenz diese Fläche (30) einstellbar ist.

4. Einrichtung nach einem der vorhergehenden Ansprüche, mit einer geschlossenen Kühlmittelkreislaufeinrichtung (5), in welcher das zerstäubte Kühlmittel (41) nach dem Auftreffen auf die zu kühlende Fläche (30) gesammelt und in den Behälter (40) rückgeführt wird.

5. Einrichtung nach Anspruch 4, wobei die Kühlmittelkreislaufeinrichtung (5) eine Kühlfläche (50) aufweist, an der Kühlmittel (412), das an der zu kühlenden Fläche (30) verdampft ist, kondensiert.

6. Einrichtung nach einem der vorhergehenden Ansprüche, wobei die zu kühlende Fläche (30) eine heiß werdende Fläche einer um eine Röhre (2) zur Aufnahme eines zu untersuchenden Patienten rotierenden elektronischen Einrichtung zur elektrischen Energieversorgung der Röntgenstrahlquelle eines Computer-Tomographen ist.

7. Einrichtung nach einem der vorhergehenden Ansprüche, wobei die zu kühlende Fläche (30) eine heiß werdende Fläche eines Targets einer um eine Röhre (2) zur Aufnahme eines zu untersuchenden Patienten rotierenden Röntgenstrahlquelle eines Computer-Tomographen ist.

## Claims

1. Device (4) for cooling a surface (30) that rotates about an axis (1) and that faces the axis (1),
- with a container (40) for a liquid coolant (41) that is disposed between the rotation axis (1) and the surface (30) for a co-rotation with the surface (30), and
- with at least one atomizer nozzle (42) of the container (40) which faces the surface (30) and from which the coolant (41) is discharged during rotation of the container (40) due to the centrifugal force (F) acting upon the coolant (41) in the container (40) in the form of an atomized jet (43) that strikes the surface (30).

2. Device according to claim 1, the atomized jet (43) striking the surface to be cooled (30) obliquely at an angle (α).

3. Device according to claim 2, the angle (α) at which the atomized jet (43) obliquely strikes the surface to be cooled (30) being selectable as a function of the rotational frequency of this surface (30).

4. Device according to one of the preceding claims, comprising a closed-loop coolant device (5) in which the vaporized coolant (41) is collected after striking the surface to be cooled (30) and returned to the container (40).

5. Device according to claim 4, the closed-loop coolant device (5) featuring a cooling surface (50) on which coolant (412) which has vaporized on the surface to be cooled (30) condenses.

6. Device according to one of the preceding claims, the surface to be cooled (30) being a surface that becomes hot of an electronic device for supplying electrical power to the X-ray source of a computer tomograph, said electronic device rotating about a tube (2) for accommodating a patient to be examined.

7. Device according to one of the preceding claims, the surface to be cooled (30) being a surface that becomes hot of a target of an X-ray source of a computer tomograph rotating about a tube designed to accommodate a patient to be examined.

## Revendications

1. Dispositif (4) pour refroidir une surface (30) laquelle est en rotation autour d'un axe (1) et tournée vers cet axe (1),
- comprenant un réservoir (40) de réfrigérant liquide (41), qui est disposé entre l'axe de rotation (1) et la surface (30) de manière à être entraîné en rotation avec la surface (30), et
- comprenant au moins une buse de pulvérisation (42) du récipient (40), laquelle est tournée vers la surface (30) et de laquelle sort le réfrigérant (41) pendant la rotation du réservoir (40) en raison de la force centrifuge (F) agissant sur le réfrigérant (41) dans le réservoir (40) sous forme de jet de pulvérisation (43) projeté sur la surface (30).

2. Dispositif selon la revendication 1, dans lequel le jet de pulvérisation (43) est projeté obliquement selon un angle (α) sur la surface (30) à refroidir.

3. Dispositif selon la revendication 2, dans lequel l'angle (α) sous lequel le jet de pulvérisation (43) est projeté obliquement sur la surface (30) à refroidir est réglable en fonction de la fréquence de rotation de cette surface (30).

4. Dispositif selon l'une des revendications précédentes, comprenant un système de recyclage de réfrigérant en circuit fermé (5) dans lequel le réfrigérant (41) pulvérisé est collecté après la projection sur la surface (30) à refroidir (30) et reconduit dans le réservoir (40).

5. Dispositif selon la revendication 4, dans lequel le système de recyclage de réfrigérant (5) comporte une surface de refroidissement (50) sur laquelle se condense le réfrigérant (412) qui s'est évaporé sur la surface (30) à refroidir.

6. Dispositif selon l'une des revendications précédentes, dans lequel la surface (30) à refroidir est une surface chauffante d'un dispositif électronique en rotation autour d'un tube (2) apte à recevoir un patient à examiner et destiné à l'alimentation en énergie électrique de la source de rayons X d'un tomographe commandé par ordinateur.

7. Dispositif selon l'une des revendications précédentes, dans lequel la surface (30) à refroidir est une surface chauffante d'une cible d'une source de rayons X en rotation autour d'un tube (2) apte à recevoir un patient à examiner d'un tomographe commandé par ordinateur.
